# EUROPÄISCHE PATENTSCHRIFT

(11) **EP 3 654 939 B1**
(45) Veröffentlichungstag und Bekanntmachung des Hinweises auf die Patenterteilung: **01.09.2021**
(21) Anmeldenummer: 18743775.1
(22) Anmeldetag: 19.07.2018
(51) Int. Cl.: A61K 9/00, A23K 40/35, A23K 10/16, A23K 10/30, A23K 20/174, A23K 50/10

(54) **BOLUS ZUR EINGABE IN DIE VORMÄGEN VON WIEDERKÄUERN UND VERFAHREN ZUR HERSTELLUNG DES BOLUS**
BOLUS FOR ADMINISTRATION TO THE FORESTOMACHS OF RUMINANTS AND METHOD FOR PRODUCING THE BOLUS
BOLUS DESTINÉ À ÊTRE ADMINISTRÉ DANS LES PRÉ-ESTOMACS DE RUMINANTS ET PROCÉDÉ DE PRÉPARATION DU BOLUS

(30) Priorität: 20.07.2017 DE 102017212520
(43) Veröffentlichungstag der Anmeldung: 27.05.2020
(73) Patentinhaber: Stumpe, Ulf-Michael, 16269 Wriezen (DE)
(72) Erfinder: Stumpe, Ulf-Michael, 16269 Wriezen (DE)
(74) Vertreter: Patentanwälte Bressel und Partner mbB
(86) Internationale Anmeldenummer: PCT/EP2018/069643
(87) Internationale Veröffentlichungsnummer: WO 2019/016321

(56) Entgegenhaltungen:
- EP-A1- 2 440 213
- DE-A1-102009 015 558
- US-A- 5 869 083
- US-A1- 2013 136 827

## Beschreibung

Die Erfindung betrifft einen Bolus, der zur Eingabe in die Vormägen von Wiederkäuern verwendet wird. Ferner betrifft die Erfindung ein Verfahren zur Herstellung des Bolus.

Es ist bekannt, dass Tiere zur Mast und zur Gewinnung von Milch auf einem energetisch sehr hohen Niveau gefüttert werden. Die Energiedichte des verwendeten Futters ist also im Allgemeinen deutlich höher, als die des natürlichen Futters, welches die Tiere in der Natur zu sich nehmen. Insbesondere gilt dies für Mast- und Milchrinder, aber auch für Schafe und Ziegen, die zur Gewinnung von Milch und Fleisch gehalten werden. Mithilfe von hochenergetischem Futter wird den Maßstäben der modernen Milch- und Mastviehhaltung Rechnung getragen.

Insbesondere wird mithilfe des hochenergetischen Futters Energie für Wachstumsprozesse und für die Produktion von Milch bereitgestellt. Die Verwendung des hochenergetischen Futters führt jedoch dazu, dass die physiologischen Vormagenfunktionen von Wiederkäuern nicht in einem Maße durchgeführt werden können, wie es im Hinblick auf die Tiergesundheit erforderlich wäre. Eine tiergerechte, natürliche Vormagenfunktion ist deshalb kaum möglich. Eine hauptsächliche Ursache hierfür ist, dass der Anteil von Rohfaser im Leistungsfutter im Vergleich zum natürlichen Futter der Tiere sehr gering ist. Ein tiergerechter Anteil von Rohfaser im Futter ist für eine tiergerechte Vormagenfunktion notwendig, da sonst eine Übersäuerung des Vormagensystems aufgrund freischwimmender Milchsäurebakterien eintreten kann. Die Auswirkungen auf die Tiergesundheit sind erheblich: Die Vormagenflora verändert sich, die Fresslust, die Wiederkauaktivität und die Vormagenaktivität der Tiere sinken stark ab, das Tier befindet sich in einem erkrankten Zustand, der lebensbedrohlich werden kann. Zudem kommt es aufgrund der Übersäuerung zur negativen Beeinflussung der immunologischen Leistungsfähigkeit.

In DE 10 2009 015 558 A1 ist ein Bolus zum Eingeben in die Vormägen von Wiederkäuern, der Rohfaser enthält, offenbart. Mithilfe von Rohfaser wird die Vormagenperistaltik unterstützt, wodurch die Wiederkauaktivität und die Umwälzung der Futterbestandteile im Vormagensystem verbessert werden.

Die im Bolus enthaltenen Substanzen, insbesondere die Rohfaser, dienen dazu, eine Grundlage zu schaffen, um die natürlichen Verdauungsvorgänge insbesondere eines bereits erkrankten Tieres wiederherzustellen und/oder zu fördern. Da eine Übersäuerung des Vormagens häufig eine Entzündung mit sich führt, vermeidet es das erkrankte Tier, strukturwirksame Rohfasern, zum Beispiel in Form von Heu oder Gras, selbst zu sich zu nehmen, da die Enden der Rohfaser, zum Beispiel in Form von Halmen, sonst schmerzhaft auf die entzündete Innenhaut des Vormagens wirken. "Strukturwirksam" in diesem Zusammenhang bedeutet, dass die Rohfaser aufgrund ihrer Form, Größe und Struktur mechanisch aktivierend und anregend auf die Verdauungsvorgänge des Vormagens wirkt. Den Unwillen des Tieres, strukturwirksame Rohfasern zu sich zu nehmen und so die zur Heilung notwendigen natürlichen Verdauungsvorgänge zu reaktivieren, überwindet der Bolus und trägt somit zur Linderung von Krankheitssymptomen und/oder zur Heilung des erkrankten Tieres bei.

Rohfaser, insbesondere in Form von Halmen, stimuliert den Vormagen, indem mechanische Reize an den Wänden des Vormagens entstehen. Durch diese Stimulation werden die Tiere zum Wiederkäuen angeregt. Dies hat zur Folge, dass der beim Wiederkäuen produzierte Speichel zur Regulation acider Milieuzustände im Vormagensystem beiträgt und so einer Übersäuerung entgegenwirkt.

Zur Umhüllung des Bolus wird in DE 10 2009 015 558 B4 ein Bolusmantel vorgeschlagen. Es wird vorgeschlagen, dass der Bolusmantel aus wasserlöslichen Materialien, zum Beispiel in Form von Kapseln, oder auch gelatinehaltigen, cellulosehaltigen oder eiweißhaltigen Produkten besteht.

Aus GB 2 353 707 A ist ein Bolus für Wiederkäuer bekannt, der teilweise oder vollständig mit einem Material beschichtet ist, das unlöslich oder kaum löslich in Wasser ist. Im Gebrauch wird die Beschichtung von dem Bolus fortschreitend durch Abrieb durch die Retikulo-Pansen entfernt.

Ein Bolus nach dem genannten Stand der Technik wird zur weiteren Verwendung unbrauchbar, sobald der Mantel oder die Beschichtung ein Loch oder einen Riss aufweist, zum Beispiel entstanden während eines erfolglosen Einbringvorgangs in das Maul des Tieres oder während eines Schluckvorgangs. Hierin liegt weiterhin die Gefahr, dass Bestandteile des Bolus sich im Maul- oder im Rachenbereich des Tieres unkontrolliert ausbreiten können und somit auch in die Luftröhre gelangen können. Dies gilt insbesondere bei einem Bolus, dessen Bestandteile nicht oder nur locker miteinander verbunden sind, so auch im Falle eines Bolus, der lediglich aus Rohfaser besteht.

Die Aufbringung eines Mantels oder einer Beschichtung verkompliziert weiterhin die Produktionsverfahren für Boli. Dies gilt wiederum insbesondere für solche Boli, die Bestandteile enthalten, welche nicht oder nur locker miteinander verbunden sind.

Insbesondere können dies im Ursprungszustand separierte Rohfasern sein. Eine Formgebung der nicht oder nur locker miteinander verbundenen Bestandteile während der Herstellung zum Zweck der nachfolgenden Aufbringung eines Mantels oder einer Beschichtung ist schwierig.

Der Erfindung liegt die Aufgabe zugrunde, einen Bolus, der Rohfaser enthält, so auszuführen, dass er die oben genannten Probleme nicht aufweist, insbesondere ohne eine Ummantelung oder eine Beschichtung und insbesondere vor einem und während eines Schluckvorgangs eines Tieres.

Ein Grundgedanke der vorliegenden Erfindung ist, die Rohfaser mit einem Bindemittel zu vermengen, sodass die Bestandteile der Rohfaser miteinander verbunden sind, grundsätzlich sowohl an der Oberfläche als auch im Inneren des Bolus.

Mit einem Bolus der Erfindung ist es möglich, die Futteraufnahme durch das Tier zu stabilisieren bzw. zu stimulieren, welches dazu führt, dass die Rohfaserkonzentration in einem physiologischen Maße zur Verfügung stehen soll. Beim Entgleisen der Vormagenfermentation entstehen einige negative systemische Stoffwechselsituationen, welche wiederrum nicht mechanisch, sondern pathophysiologisch zur metabolischen Verminderung der Fresslust führen. Dem kann mit einem erfindungsgemäßen Bolus entgegen gewirkt werden.

Durch die Formstabilisierung kann das unkontrollierte Aufquellen der Rohfaseranteile verhindert werden, wenn die Luftfeuchtigkeit oder Feuchtigkeit der Maulhöhle auf den Rohfaserbolus einwirkt.

Daher wird zur Lösung der ersten Aufgabe ein Bolus nach Schutzanspruch 1 offenbart.

Die Teile der Rohfaser sind als Anteil des Gemenges aus Rohfaser und dem mindestens einem Bindemittel aufgrund des mindestens einen Bindemittels miteinander verbunden. Dies ist einerseits vorteilhaft, da sich das Produktionsverfahren des Bolus, das später genauer erörtert wird, vereinfacht. So entfällt der komplizierte Schritt, auf nicht oder nur locker verbundene Bestandteile des Bolus eine Beschichtung oder einen Mantel aufbringen zu müssen. Andererseits ergibt sich der Vorteil, dass eine äußere Beschädigung, zum Beispiel ein Loch oder ein Riss, den Bolus nicht unbrauchbar werden lässt, da sich trotz der Beschädigung die Form und die Struktur des Bolus nicht oder nicht wesentlich verändern. Derartige Beschädigungen können insbesondere während eines Einbringvorgangs in das Maul des Tieres und während eines Schluckvorgangs entstehen.

In diesem Zusammenhang steht ein weiterer wesentlicher Vorteil: Die Gefahr, dass der Bolus sich während eines Eingabevorgangs in das Maul des Tieres, der mittels handelsüblicher Eingabehilfen erfolgt, oder während eines Schluckvorgangs zersetzt oder beschädigt wird, ist auf ein Minimum reduziert. Es ist somit sehr unwahrscheinlich, dass Bestandteile des Bolus sich im Maul- oder im Rachenbereich des Tieres unkontrolliert ausbreiten und somit auch in die Luftröhre gelangen können. Die negativen Konsequenzen hieraus, die einerseits darin bestehen, dass für das Tier wertvolle Bestandteile des Bolus den Vormagen nicht erreichen, und andererseits, dass Atembeschwerden des Tieres und/oder gar Luftnot bis hin zum Ersticken auftreten, sind weitgehend ausgeschlossen.

Ein weiterer Vorteil liegt darin, dass das Gemenge aus Rohfaser und mindestens einem Bindemittel dazu führt, dass der Bolus leicht durch die Speiseröhre des Tieres transportiert werden kann. Dies gilt insbesondere, wenn mindestens ein Bindemittel und eine Bindemittelmenge verwendet wird, das/die zum Beispiel zu einer glatten und klar definierten und/oder gut rutschenden Oberfläche des Bolus führt, zum Beispiel Fett. Weitere mögliche Bindemittel werden unten erwähnt.

Der Begriff "Rohfaser" kann in diesem Zusammenhang bezeichnend sein für natürliche faserige Stoffe, dies schließt insbesondere Pflanzenteile mit ein wie später noch genauer erläutert wird. Auch können unter "Rohfaser" künstliche faserige Stoffe verstanden werden, zum Beispiel synthetische faserförmige Cellulose.

Das mindestens eine Bindemittel kann grundsätzlich ein strukturgebender Stoff oder mehrere strukturgebende Stoffe sein. Unter "strukturgebend" ist zu verstehen, dass durch das Bindemittel der Bolus eine stabile Form beibehält oder behält, bis er an dem gewünschten Ort im Vormagen eines Wiederkäuers zersetzt wird.

Das Bindemittel kann dem Verbund aus Rohfaser und Bindemittel Formbeständigkeit verleihen. Das heißt insbesondere, dass der Stoff/die Stoffe bei Einwirkung durch äußerlich ausgeprägten Druck, gegebenenfalls unter dem Einfluss einer gegenüber der Umgebung erhöhten Temperatur, keine oder nur wenige Fehlstellen innerhalb des Bolus in Form von Rissen oder ähnlichen Defekten bildet/bilden und sich leicht und fest mit sich selbst verbindet/verbinden.

Es kann mithilfe des mindestens einen Bindemittels eine Strukturbeständigkeit des Bolus auch in ungünstigen Lagerungsbedingungen, zum Beispiel einer hohen Feuchtigkeit, einer hohen Trockenheit oder Hitze, erreicht werden. Eine derartige Strukturbeständigkeit könnte durch die alleinige Verwendung von ausschließlicher Rohfaser ohne das mindestens eine Bindemittel nicht erreicht werden.

Das mindestens eine Bindemittel ist nicht im Sinne einer Ummantelung zu verstehen, sondern als ein Stoff, der sich verteilt, vorzugsweise gleichmäßig verteilt und sich in einem überwiegenden Teil des Volumens, vorzugsweise im gesamten Volumen des Bolus wiederfindet. Dies schließt die Außenflächen des Bolus und alle inneren Bereiche grundsätzlich mit ein. Anders ausgedrückt erstreckt sich das Gemenge aus Rohfaser und Bindemittel bis in das Innere des Bolus, vorzugsweise über das gesamte Volumen des Bolus. Vorzugsweise weist der Bolus ein gleichmäßiges oder im wesentlichen gleichmäßiges Gemenge aus Rohfaser und Bindemittel auf.

Durch das Bindemittel werden einzelne Rohfasern oder Rohfaserbestandteile miteinander verbunden.

Rohfaser oder Rohfaserbestandteile können vollständig oder teilweise von Bindemittel umgeben sein, bzw. vollständig oder teilweise in Bindemittel eingebettet sein. An der Außenseite des Bolus können Rohfasern oder Rohfaserbestandteile teilweise aus der Oberfläche hervor stehen.

Das Verhältnis von Rohfaser zu Bindemittel kann variabel gewählt werden. Das Verhältnis wird vorzugsweise so gewählt, dass ein strukturbeständiger Bolus erhalten wird. Insbesondere wird das Verhältnis vorzugsweise so gewählt, dass keine nicht durch Bindemittel mit anderen Rohfasern verbundenen Rohfasern vorliegen.

Der Bolus kann Hohlräume aufweisen, die weder mit Rohfaser noch mit Bindemittel gefüllt sind.

Zusätzlich zum mindestens einen Bindemittel kann der Bolus einen Bolusmantel aufweisen. Der Bolusmantel kann aus einem Stoff bestehen, der sich in Flüssigkeit, so zum Beispiel im Vormagen, zersetzt. Der Bolusmantel kann aus beliebigen tierverträglichen Stoffen bestehen, insbesondere aus Cellulose, Eiweißen, Gelatine oder stärkehaltigen Substanzen. Auch ein Bolusmantel aus einem fetthaltigen Stoffwechselpräparat zur Verabreichung bei Energiemangelzuständen, also einem Ergänzungsfuttermittel, ist denkbar, zum Beispiel "Ket 40", eine Marke des Herstellers "ufamed AG". In dieser Form bietet der Bolusmantel zusätzlich eine energetische Komponente.

Weiterhin kann der Bolus und/oder das mindestens eine Bindemittel mindestens eine weitere Substanz aufweisen, die der Tiergesundheit förderlich ist oder/und eine sonstige Funktion besitzt. Verschiedene Beispiele hierfür finden sich in Ausgestaltungen der Erfindung, die weiter unten vorgeschlagen werden.

Durch die Möglichkeit, dass der Bolus, insbesondere das mindestens eine Bindemittel mindestens eine weitere Substanz aufweist, können neben dem Mangel an strukturwirksamen Rohfaseranteilen und dessen Folgen weitere krankhafte Zustände eines Tiers geheilt oder gelindert werden. Dies betrifft insbesondere Übersäuerungen (Azidosen), Stoffwechselstörungen, Fermentationsstörungen und/oder ein mikrobielles Ungleichgewicht. Ebenso können auch andere Krankheiten, zum Beispiel Ketosen oder Milchfieber, behandelt werden oder verschiedene Defizite. Hierzu zählen allgemein Energiedefizite, Mineralstoffdefizite (insbesondere Defizite von Magnesium, Phosphor, Kalzium oder Kalium), Vitamindefizite und Defizite von Spurenelementen.

Vorteilhaft sind solche Substanzen, die nicht pharmakologisch wirksam sind und so keine Sperrzeiten auf die Milch und/oder auf essbares Gewebe mit sich ziehen. Nichtsdestotrotz ist die Gabe von pharmakologischen Substanzen durch den Bolus zum Beispiel mithilfe des mindestens einen Bindemittels als Träger ebenso möglich. Dies gilt insbesondere für solche pharmakologischen Substanzen, die appetitanregend wirken.

Wie später noch genauer erläutert wird, kann das mindestens eine Bindemittel selbst eine der Tiergesundheit förderliche Funktion haben. Wird für das mindestens eine Bindemittel Eiweiß, Zucker oder Fett verwendet, bietet es gleichsam, zusätzlich zu den beschriebenen Funktionen, eine energetische Funktion.

Sobald der Bolus den Vormagen erreicht hat, soll sich die Struktur des Bolus auflösen. Die Bestandteile des Bolus sollen sich hierbei im Vormagen lösen. Der Auflösungsvorgang kann schnell und zeitlich dem Schluckvorgang naheliegend erfolgen. Auch möglich ist, dass sich die Bestandteile des Bolus langsamer lösen, insbesondere in einer Geschwindigkeit, die durch den Auflösungsvorgang des mindestens einen Bindemittels bestimmt wird. So kann eine zeitlich kontinuierliche Freisetzung der im Bolus enthaltenen Stoffe, die der Tiergesundheit förderlich sind, erfolgen. Die Stimulation der Fresslust eines Tieres, ausgelöst insbesondere durch im Bolus enthaltene Rohfaser, verläuft somit nicht ungesteuert.

Grundsätzlich kann der Bolus nicht nur für Rinder, sondern für jegliche Wiederkäuer verwendet werden, zum Beispiel Ziegen, Schafe und auch Wildwiederkäuer, so zum Beispiel Rentiere, Rehe und Hirsche. Die Größe des Bolus kann hierbei variieren, insbesondere tierartengerecht variieren. Typische Abmaße für Rinder sind: Durchmesser 35-40 mm, Länge 160 mm; typische Abmaße für Schafe und Ziegen sind: Durchmesser 15 mm, Länge 70 mm.

Der Bolus besitzt vorzugsweise die Grundgestalt einer Kapsel, das heißt, dass er zylinderförmig ist und nach außen abgerundete Enden besitzt. Die Grundgestalt bezeichnet hier die grundsätzliche Form des Bolus, wie sie zum Beispiel durch ein Formwerkzeug während eines Bearbeitungsprozesses vorgegeben sein könnte. Das heißt, dass Anteile des Bolus durchaus über die Grundgestalt hinausragen können, wie auch weiter unten beschrieben.

Die Grundgestalt einer Kapsel bietet grundsätzlich, insbesondere aufgrund der abgerundeten Enden, gute Gleiteigenschaften. Die Grundgestalt einer Kapsel ist jedoch nicht die einzige mögliche Grundgestalt. Der Bolus kann insbesondere auch eine Grundgestalt eines Ellipsoids, eines Quaders, eines Würfels, einer Kugel oder eines Zylinders besitzen. Dies schließt jegliche andere Formgebung nicht aus.

In der erfindungsgemäßen Ausgestaltung macht ein Gemenge aus Rohfasern und dem mindestens einen Bindemittel einen Anteil von mindestens 20 % des Volumens des Bolus aus. Vorzugsweise hat das Gemenge aus Rohfasern und mindestens einen Bindemittel einen Anteil von mindestens 30 % des Volu mens (Vol-%) des Bolus und besonders vorzugsweise einen Anteil von mindestens 40 Vol-%. Weitere alternative Untergrenzen ergeben sich bei einem Anteil von mindestens 50 Vol-%, von mindestens 60 Vol-%, von mindestens 70 Vol-%, von mindestens 80 Vol-% und von mindestens 90 Vol-%. Eine Obergrenze, die mit allen genannten Untegrenzen kombiniert werden kann, ist 100 Vol.-%

In einer weiteren vorteilhaften Ausgestaltung besteht der Bolus im Wesentlichen aus dem Gemenge aus Rohfasern und dem mindestens einen Bindemittel. Das heißt insbesondere, dass andere Bestandteile und/oder Hohlräume nur einen geringen, insbesondere höchstens einstelligen (vorzugsweise weniger als 5 Vol-%), prozentualen Volumenanteil des Bolus einnehmen. Die genannten vorteilhaften Effekte auf die Vormagengesundheit bzw. auf die Gesundheit des Wiederkäuers ergeben sich analog in besonders ausgeprägter Form. Demgemäß ist auch ein Bolus, der im Wesentlichen oder vollständig aus dem Gemenge aus Rohfasern und dem mindestens einen Bindemittel besteht, möglich. Der Begriff "im Wesentlichen" bedeutet insbesondere einen Anteil des Gemenges von mindestens 95 % des Volumens (Vol-%) des Bolus, oder mindestens 96 Vol-%, oder mindestens 97 Vol-%, oder mindestens 98 Vol-%, wobei eine Obergrenze für den Begriff "im Wesentlichen" vorzugsweise 99,9 Vol-% ist, oder 99,99 Vol-%. Vollständig bedeutet einen Anteil von 100 Vol-%.

Der Volumenanteil der Rohfasern in dem Gemenge aus Rohfasern und dem mindestens einen Bindemittel beträgt vorzugsweise mindestens 20% des Volumens (Vol-%) des Gemenges, vorzugsweise mindestens 40 Vol-%. Weitere alternative Untergrenzen ergeben sich bei einem Anteil von mindestens 50 Vol-%, von mindestens 60 Vol-%, von mindestens 70 Vol-%, von mindestens 80 Vol-%, und von mindestens 90 Vol-%. Der Volumenanteil der Rohfasern in dem Gemenge aus Rohfasern und dem mindestens einen Bindemittel kann als Obergrenze 95 Vol-%, 96 Vol-%, 97 Vol-% 98 Vol-% oder 99 Vol-% des Gemenges betragen, wobei jede der genannten Obergrenzen mit jeder der genannten Untergrenzen beliebig kombinierbar ist.

Insbesondere bei den oben genannten Abmaßen des Bolus ergeben sich spürbare vorteilhafte Effekte auf die Vormagengesundheit bzw. auf die Gesundheit des Wiederkäuers insbesondere, wenn eine ausreichende Menge von Rohfasern in den Vormagen eingebracht wird. In der erfindungsgemäßen Ausführungsform werden mindestens 20 % des Volumens des Bolus durch das Gemenge aus Rohfasern und dem mindestens einen Bindemittel eingenommen. Höhere Anteile sind insbesondere wegen des höheren Anteils an Rohfasern in diesem Zusammenhang besonders vorteilhaft.

Bei einer ausgewachsenen Kuh kann der Vormagen ein Volumen von mehr als 100 I erreichen. Zur Erhöhung der Wirksamkeit ist in der Praxis eine Gabe von mehreren Boli in kurzen Abständen oder nacheinander möglich. Auch ist eine regelmäßige Gabe von Boli zur Erhöhung der Wirksamkeit möglich.

In einer weiteren vorteilhaften Ausgestaltung, die alternativ oder zusätzlich zu anderen hier erwähnten Ausgestaltungen Anwendung finden kann, machen die Rohfasern (nur die Rohfasern, ohne Bindemittel) einen Anteil von mindestens 20 % des Volumens (Vol-%) des Bolus aus. Vorzugsweise haben Rohfasern einen Anteil von mindestens 30 Vol-% des Bolus und besonders vorzugsweise einen Anteil von mindestens 40 Vol-%. Weitere alternative Untergrenzen ergeben sich bei einem Anteil von mindestens 50 Vol-%, von mindestens 60 Vol-%, von mindestens 70 Vol-%, von mindestens 80 Vol-% und von mindestens 90 Vol-%. Auch ein Bolus, der im Wesentlichen aus Rohfasern besteht, sodass das Bindemittel nur einen höchstens einstelligen prozentualen Anteil des Volumens des Bolus einnimmt (vorzugsweise weniger als 5 Vol-%), ist möglich. Eine Obergrenze für den Volumenanteil Rohfaser in dem Bolus kann 99 Vol-% sein, oder 98 Vol-%, oder 97 Vol-%, oder 96 Vol-%, oder 95 Vol-%, wobei jede der genannten Obergrenzen mit jeder der genannten Untergrenzen beliebig kombinierbar ist. Die genannten vorteilhaften Effekte auf die Vormagengesundheit bzw. auf die Gesundheit des Wiederkäuers ergeben sich analog wie oben beschrieben.

In einer weiteren vorteilhaften Ausgestaltung weist das mindestens eine Bindemittel hydrophobe Eigenschaften auf. Dies dient dazu, ein Aufquellen des Bolus und/oder einzelner Bestandteile des Bolus, insbesondere Rohfaser, aufgrund von Feuchtigkeit zu vermeiden. Dies bezieht sich insbesondere auf Feuchtigkeit, die während der Lagerung des Bolus in der Lagerungsumgebung auftritt, aber auch auf Feuchtigkeit im Maulbereich und im Rachenbereich des Tieres. Das mindestens eine Bindemittel mit hydrophoben Eigenschaften dient also auch dazu, zu gewährleisten, dass der Bolus während des Schluckvorgangs seine Struktur behält und nicht zerfällt, also während der Lagerung und während des Schluckvorgangs ein ausreichendes Maß an Strukturstabilität aufweist. Dies ist dadurch gewährleistet, dass aufgrund der hydrophoben Eigenschaften des mindestens einen Bindemittels während des Schluckvorgangs keine/nur eine geringe Wasseraufnahme des Bolus stattfindet. Dies geht einher mit dem Ziel, dass ein Zersetzungsvorgang des Bolus erst im Vormagen des Tieres beginnen soll.

Zur Ausbildung der hydrophoben Eigenschaften kann mittels eines Plasmas eine Bearbeitung an der Oberfläche des Bolus und/oder des mindestens einen Bindemittels oder eines weiteren hinzuzufügenden Stoffs vorgenommen werden, um die hydrophoben Eigenschaften zu erzielen.

In einer weiteren Ausführungsform weist der Bolus als Bestandteil mindestens eine Substanz auf, oder besteht aus einer Substanz, die ausgewählt ist aus der Gruppe bestehend aus Fetten, Proteinen, Kohlehydraten, Salzen, synthetischen hochmolekularen Stoffen und/oder Mineralstoffen und deren Verbindungen. Insbesondere weist das Bindemittel mindestens einen dieser Bestandteile auf oder besteht daraus. Mindestens einer der genannten Bestandteile kann als weiterer Bestandteil zugesetzt sein, wenn er nicht schon im Bindemittel enthalten ist. Der mindestens eine genannte Bestandteil kann in beliebiger Form innerhalb des Bolus vorliegen. So kann er der Rohfaser, die für den Bolus verwendet wird, in jeglicher Art und Weise hinzugefügt sein, oder dem mindestens einen Bindemittel, oder dem Gemenge aus Rohfaser und dem mindestens einen Bindemittel, oder jeglichem anderen Bestandteil des Bolus zugefügt sein. Der mindestens eine genannte Bestandteil kann auch in einer separaten Einheit oder in einem separaten Bereich innerhalb oder an der Außenhaut des Bolus vorliegen, zum Beispiel in einem Hohlraum oder in mindestens einer separaten Kapsel, die dem Bolus in seinem Inneren oder an seiner Außenhaut hinzugefügt ist, oder in einem sonstigen Raum innerhalb des Bolus oder außerhalb am Bolus. Diese Ausführungsform hat den Vorteil der Förderung von fermentativer Arbeit von Mikroorganismen, welche als Bestandteil dem Bolus beigefügt werden oder im Magen oder Vormagen des Tieres schon vorhanden sein können. Verfügbarkeit von Fetten, Eiweißen, Kohlenhydraten, und/oder nachfolgend noch genannter Spurenelemente dient dem Ziel einer schnellen Genesung der Vormagenflora. Auch ein fetthaltiges Stoffwechselpräparat zur Verabreichung bei Energiemangelzuständen, also ein Ergänzungsfuttermittel, ist denkbar. Es kann sich hierbei wiederum zum Beispiel um "Ket 40", eine Marke des Herstellers "ufamed AG" handeln.

Salze können organische oder anorganische Salze sein. Zum Zweck der Verwendung als Bindemittel können die Salze geschmolzen, erhärtet und/oder verpresst werden, gegebenenfalls unter Temperatureinwirkung.

Geeignete Salze sind beispielsweise Salze mit den Kationen Calcium, Kalium, Magnesium, Natrium, und den Anionen Chlorid, Phosphat, Hydrogenphosphat, Sulfat, Sulfid, Carbonat, Hydrogencarbonat, Nitrit, Nitrat oder Fluorid, wobei diese Kationen und Anionen in beliebiger Weise kombiniert werden können.

Einige Salze, zum Beispiel Calciumchlorid oder Kaliumchlorid, werden bei einem Pressvorgang innerhalb eines Herstellungsverfahrens zu einem strukturell sehr festen und dichten Verbund und eignen sich deshalb besonders gut zur Verwendung als das mindestens eine Bindemittel.

Es ist vorteilhaft, wenn beim Herstellen (insbesondere bei einem Pressvorgang) des Bolus, insbesondere in Bezug auf die Rohfaser, ein Schwammeffekt aufgrund eines Aufquellens vermieden werden kann. Dies wird dadurch erreicht, dass das mindestens eine Bindemittel sich zumindest anteilig aus einem oder mehreren der genannten Stoffe, sofern für eine Vermeidung des genannten Aufquelleffekts geeignet, zusammensetzt. Hierzu sind zum Beispiel Fette, Proteine und Kohlenhydrate geeignet, die gleichsam noch eine Bedeutung als Energielieferant für das Tier haben und als Starter für Verdauungsvorgänge fungieren können. Zur Erklärung: Zur Arbeitsfähigkeit der Mikroorganismen im Vormagen sind zum Beispiel Fette, Proteine und Kohlenhydrate in Kombination mit Rohfaser besonders vorteilhaft.

Die im vorherigen Absatz genannten Stoffe sind weiterhin dazu geeignet, Rohfaser zu binden bzw. zu verkleben bzw. zu fixieren. Hierzu zählen auch einige synthetische, hochmolekulare Stoffe. Solche Stoffe sind zum Beispiel Polymere. Es können sowohl im Verdauungssystem des Wiederkäuers abbaubare Stoffe als auch nicht dort abbaubare Stoffe verwendet werden. Besonders bevorzugt sind Stoffe die nicht im Verdauungssystem des Wiederkäuers abbaubar sind, den Körper des Tieres also in chemisch inerter Weise durchlaufen.

Einige der genannten Stoffe sind weiterhin dazu geeignet, Mikroorganismen in einem Vormagen mit Energie zu versorgen. Dies sind zum Beispiel Fette, Kohlehydrate, Proteine und Mineralstoffe (zum Beispiel Calcium, Chlor, Kalium, Magnesium, Natrium, Phosphor, Schwefel, Hydrogencarbonate, Sulfate, Nitrate, Fluoride).

Der Bolus kann eine Puffersubstanz enthalten, womit ein pH-Wert im Vormagen des Wiederkäuers konstant gehalten werden kann. Ein Beispiel hierfür ist ein Hydrogencarbonat.

In einer weiteren Ausgestaltung weist die im Bolus verwendete Rohfaser Pflanzenteile auf oder besteht aus Pflanzenteilen. Pflanzenteile stellen das natürliche Futter von Wiederkäuern dar und eignen sich aufgrund der enthaltenen Cellulose, die im Vormagen umgesetzt wird, zur Aktivierung und Unterstützung der natürlichen Verdauungsvorgänge. Wie in den Abschnitten zur nächsten Ausgestaltung erklärt ist, eignen sich hierzu insbesondere Heu oder Stroh. Hierneben sind jedoch prinzipiell auch andere Arten von verarbeiteten oder unverarbeiteten Pflanzenteilen zur Verwendung im Bolus geeignet, sofern sie die natürlichen Verdauungsvorgänge aktivieren bzw. unterstützen und tierverträglich sind.

In einer weiteren vorteilhaften Ausgestaltung sind die Pflanzenteile Heu und/oder Stroh. Pflanzenteile, bestehend aus Heu und/oder Stroh, sind besonders gut zur Aktivierung und Unterstützung der natürlichen Verdauungsvorgänge geeignet, da sie, neben ihrem Cellulosegehalt, durch mechanische Reize an den Wänden eines Vormagens die natürlichen Verdauungsprozesse stimulieren.

Das Heu und/oder das Stroh kann älteres oder jüngeres Heu und/oder älteres oder jüngeres Stroh sein, bezogen auf den Ernte- bzw. Verarbeitungsvorgang und/oder auf das Alter der geernteten/verarbeiteten Pflanze. Ist das Heu und/oder das Stroh älter, ist die Verdaubarkeit im Allgemeinen geringer, die Strukturwirksamkeit jedoch höher. Ist das Heu und/oder Stroh jünger, ist die Verdaubarkeit im Allgemeinen höher, die Strukturwirksamkeit jedoch geringer. Als Rohfaser im Bolus kann also, je nach beabsichtigtem Zweck, anteilig älteres oder jüngeres Heu und/oder anteilig älteres oder jüngeres Stroh verwendet werden.

In einer weiteren vorteilhaften Ausgestaltung beträgt die Länge der Rohfasern 3 cm bis 8 cm, vorzugsweise 5 cm bis 8 cm. Diese Länge kann beispielsweise durch Häckseln eingestellt werden. Es hat sich gezeigt, dass bei dieser Länge die im vorherigen Absatz beschriebene Stimulation in hohem Maße einsetzt. Bei der Angabe 3 cm bis 8 cm, vorzugsweise 5 cm bis 8 cm handelt es sich um kein im strengen Sinne festgesetztes Maß, sondern um eine grundsätzliche Angabe. Es ist also möglich, dass trotz der angegebenen Häcksellänge kürzere oder längere Bestandteile in der Rohfaser vorhanden sind.

Weitere mögliche Bestandteile des Bolus sind nachfolgend erwähnt. Mit diesen Bestandteilen können weitere Wirkungen erzielt werden und/oder Applikationssynergien erreicht werden. Zum Beispiel können bei der Verabreichung von nachfolgend genannten Vitaminen oder pharmakologisch wirksamen Substanzen Stichinjektionen vermieden oder verringert werden. Andere Vorteile sind an betreffender Stelle genannt.

Mit der Gabe eines oder mehrerer der zusätzlichen, nachfolgend aufgeführten Bestandteile können Rekonvalenzenssynergien mit dem Bolus in folgendem Sinne erreicht werden: Primäre oder sekundäre Erkrankungen der Vormägen führen direkt (Versagen der mikrobiologischen Flora) oder indirekt durch metabolische Störungen zur Reduktion der Futteraufnahme und der Leistungsfähigkeit des Tieres. Zudem wirken in ähnlicher Weise überschießende Entzündungsprodukte allgemein dämpfend auf die Futteraufnahme. Soll nun das oberste Ziel, die Verbesserung der Futteraufnahme, erreicht werden, können je nach Krankheitsgeschehen durch Gabe bestimmter Stoffe, wie z. B. Mineralien, insbesondere Calcium, Phosphor, Kalium, oder Vitamine, energetische Defizite etc. ausgeglichen werden bzw. überschießende Entzündungsprodukte neutralisiert werden.

Somit ist es in speziellen Aussführungsformen mit der Erfindung möglich, die negativen primären und sekundären Effekte einer entgleisten Vormagengesundheit zu kompensieren oder diesen entgegen zu wirken. Man spricht hier auch von Heilungssynergie (Rekonvaleszenzsynergie). Es kann zum einen ein Rohfaserdefizit ausgeglichen werden und weiterhin können durch einen oder mehrere der genannten weiteren Bestandteile sekundäre metabolische Krankheitserscheinungen bekämpft werden, um insbesondere die Ursache des Rohfaserdefizits zu bekämpfen. Hierdurch kann eine Synergie erzielt werden von Futteraufnahme, Stimulierung der Fresslust und ggf. Rekonvaleszenz.

### Spurenelement:

In einer weiteren vorteilhaften Ausgestaltung enthält der Bolus mindestens ein Spurenelement, insbesondere aus der Gruppe bestehend aus Kobalt, Eisen, Fluor, Jod, Kupfer, Mangan, Molybdän, Selen, Silizium, Vanadium, Zink, Chrom und deren Verbindungen. Ein solches Spurenelement kann als weiterer Bestandteil zugesetzt sein, wenn nicht das Bindemittel schon ein solches enthält. Das mindestens eine Spurenelement kann in beliebiger Form innerhalb des Bolus vorliegen. So kann es der Rohfaser, die für den Bolus verwendet wird, in jeglicher Art und Weise hinzugefügt sein, oder dem mindestens einen Bindemittel oder dem Gemenge aus Rohfaser und dem mindestens einen Bindemittel oder jeglichem anderen Bestandteil des Bolus. Das mindestens eine Spurenelement kann auch in einer separaten Einheit oder in einem separaten Bereich innerhalb oder an der Außenhaut des Bolus vorliegen, zum Beispiel in einem Hohlraum innerhalb des Gefüges des Bolus oder in mindestens einer separaten Kapsel, die dem Bolus in seinem Inneren oder an seiner Außenhaut hinzugefügt ist, oder in einem sonstigen Raum innerhalb des Bolus oder außerhalb am Bolus.

Insbesondere kann das mindestens eine Spurenelement mit dem mindestens einen Bindemittel oder/und dem Gemenge aus Rohfaser und mindestens einem Bindemittel oder/und mit mindestens einem anderen Bestandteil des Bolus vermischt sein. Dies ist einerseits hinsichtlich eines Herstellungsprozesses des Bolus von Bedeutung, denn der Herstellungsprozess ist durch ein bloßes Hinzufügen und Mischen mit dem mindestens einen Spurenelement besonders einfach. Andererseits ergibt sich der Vorteil, dass das mindestens eine Spurenelement sich mit dem mindestens einen Bindemittel in zeitlich kontinuierlicher Art und Weise, entsprechend dem Auflösungsvorgang des mindestens einen Bindemittels, im Vormagen freisetzen kann. Die zeitlich kontinuierliche Art und Weise der Freisetzung kann der Tiergesundheit förderlich sein.

Ebenso kann das Spurenelement selbst Teil des mindestens einen Bindemittels sein oder in einem Bestandteil des mindestens einen Bindemittels enthalten sein. Die im vorherigen Absatz beschriebenen Vorteile ergeben sich analog.

### Vitamin

In einer weiteren vorteilhaften Ausgestaltung enthält der Bolus mindestens ein Vitamin, insbesondere aus der Gruppe bestehend aus Vitamin A, Vitamin D, Vitamin E, Vitamin K1, Vitamin K2, Vitamin K3, Vitamin K4, Vitamin C, Vitamin B1, Vitamin B2, Vitamin B3, Vitamin B5, Vitamin B6, Vitamin B7, Vitamin B9, Vitamin B12. Ein solches Vitamin kann als weiterer Bestandteil zugesetzt sein, wenn nicht das Bindemittel schon ein solches enthält. Das mindestens eine Vitamin kann in beliebiger Form innerhalb des Bolus vorliegen. So kann es der Rohfaser, die für den Bolus verwendet wird, in jeglicher Art und Weise hinzugefügt sein, oder dem mindestens einen Bindemittel oder dem Gemenge aus Rohfaser und dem mindestens einen Bindemittel oder jeglichem anderen Bestandteil des Bolus. Das mindestens eine Vitamin kann auch in einer separaten Einheit oder in einem separaten Bereich innerhalb oder an der Außenhaut des Bolus vorliegen, zum Beispiel in einem Hohlraum oder in mindestens einer separaten Kapsel, die dem Bolus in seinem Inneren oder an seiner Außenhaut hinzugefügt ist, oder in einem sonstigen Raum innerhalb des Bolus oder außerhalb am Bolus.

Insbesondere kann das mindestens eine Vitamin mit dem mindestens einen Bindemittel oder/und dem Gemenge aus Rohfaser und mindestens einem Bindemittel oder/und mit mindestens einem anderen Bestandteil des Bolus vermischt sein. Dies ist einerseits hinsichtlich eines Herstellungsprozesses des Bolus von Bedeutung, denn der Herstellungsprozess ist durch ein bloßes Hinzufügen und Mischen mit dem mindestens einen Vitamin besonders einfach. Andererseits ergibt sich der Vorteil, dass das mindestens eine Vitamin sich mit dem mindestens einen Bindemittel in zeitlich kontinuierlicher Art und Weise, entsprechend dem Auflösungsvorgang des mindestens einen Bindemittels, im Vormagen freisetzen kann. Die zeitlich kontinuierliche Art und Weise der Freisetzung kann der Tiergesundheit förderlich sein.

Ebenso kann das Vitamin selbst Teil des mindestens einen Bindemittels sein oder in einem Bestandteil des mindestens einen Bindemittels enthalten sein. Die im vorherigen Absatz beschriebenen Vorteile ergeben sich analog.

### Gesundheits- und/oder verdauunasförderndes Mittel

In einer weiteren vorteilhaften Ausgestaltung enthält der Bolus mindestens ein gesundheits- und/oder verdauungsförderndes Mittel, insbesondere aus der Gruppe bestehend aus Magnesium und Magnesiumverbindungen, Phosphor und Phosphorverbindungen, Kalzium und Kalziumverbindungen, Kalium und Kaliumverbindungen, Hydrogencarbonaten, Kräuterauszügen, Kräuterextrakten, ätherischen Ölen und pharmakologisch wirksamen Substanzen. Ein solches gesundheits- und/oder verdauungsförderndes Mittel kann als weiterer Bestandteil zugesetzt sein, wenn nicht das Bindemittel schon ein solches enthält. Das mindestens eine gesundheits- und/oder verdauungsfördernde Mittel kann in beliebiger Form innerhalb des Bolus vorliegen. So kann es der Rohfaser, die für den Bolus verwendet wird, in jeglicher Art und Weise hinzugefügt sein, oder dem mindestens einen Bindemittel oder dem Gemenge aus Rohfaser und dem mindestens einen Bindemittel oder jeglichem anderen Bestandteil des Bolus. Das mindestens eine gesundheits- und/oder verdauungsfördernde Mittel kann auch in einer separaten Einheit oder in einem separaten Bereich innerhalb oder an der Außenhaut des Bolus vorliegen, zum Beispiel in einem Hohlraum oder in mindestens einer separaten Kapsel, die dem Bolus in seinem Inneren oder an seiner Außenhaut hinzugefügt ist, oder in einem sonstigen Raum innerhalb des Bolus oder außerhalb am Bolus.

Insbesondere kann das mindestens eine gesundheits- und/oder verdauungsfördernde Mittel mit dem mindestens einen Bindemittel oder/und dem Gemenge aus Rohfaser und mindestens einem Bindemittel oder/und mit mindestens einem anderen Bestandteil des Bolus vermischt sein. Dies ist einerseits hinsichtlich eines Herstellungsprozesses des Bolus von Bedeutung, denn der Herstellungsprozess ist durch ein bloßes Hinzufügen und Mischen mit dem mindestens einen gesundheits- und/oder verdauungsfördernden Mittel besonders einfach. Andererseits ergibt sich der Vorteil, dass das mindestens eine gesundheits- und/oder verdauungsfördernde Mittel sich mit dem mindestens einen Bindemittel in zeitlich kontinuierlicher Art und Weise, entsprechend dem Auflösungsvorgang des mindestens einen Bindemittels, im Vormagen freisetzen kann. Die zeitlich kontinuierliche Art und Weise der Freisetzung kann der Tiergesundheit förderlich sein.

Ebenso kann das gesundheits- und/oder verdauungsfördernde Mittel selbst Teil des mindestens einen Bindemittels sein oder in einem Bestandteil des mindestens einen Bindemittels enthalten sein. Die im vorherigen Absatz beschriebenen Vorteile ergeben sich analog.

### Hefe, mikrobiologische Starterkulturen

In einer weiteren vorteilhaften Ausgestaltung enthält der Bolus als Bestandteil Hefe und/oder mikrobiologische Starterkulturen.

Eine mikrobiologische Starterkultur hat die Funktion der, und dient zur, Initiierung von Verdauungsprozessen in einem Vormagen, und kann weiterhin zur Stabilisierung und Förderung der mikrobiologischen Integrität im Vormagen dienen. Ein Beispiel für eine mikrobiologische Starterkultur ist ein sogenannter Pansenstarter.

Hefe, die hier getrennt von mikrobiologischer Starterkultur genannt ist, kann eine gleiche Funktion aufweisen wie die mikrobiologische Starterkultur und dabei überschüssige, leicht lösliche Kohlenhydrate eliminieren.

Mindestens einer der genannten Bestandteile kann als weiterer Bestandteil zugesetzt sein, wenn er nicht schon im Bindemittel enthalten ist. Der mindestens eine genannte Bestandteil kann in beliebiger Form innerhalb des Bolus vorliegen. So kann er der Rohfaser, die für den Bolus verwendet wird, in jeglicher Art und Weise hinzugefügt sein, oder dem mindestens einen Bindemittel, oder dem Gemenge aus Rohfaser und dem mindestens einen Bindemittel, oder jeglichem anderen Bestandteil des Bolus zugefügt sein. Der mindestens eine genannte Bestandteil kann auch in einer separaten Einheit oder in einem separaten Bereich innerhalb oder an der Außenhaut des Bolus vorliegen, zum Beispiel in einem Hohlraum oder in mindestens einer separaten Kapsel, die dem Bolus in seinem Inneren oder an seiner Außenhaut hinzugefügt ist, oder in einem sonstigen Raum innerhalb des Bolus oder außerhalb am Bolus.

Eine mikrobiologische Starterkultur kann insbesondere aus Mikroorganismen bestehen. Die Mikroorganismen können insbesondere eine oder mehrere der folgenden Eigenschaften haben: Sie können aufgrund spezifischer Eigenschaften selektiert sein. Die Mikrooranismen können vermehrungsfähig sein. Die Mikroorganismen können bei fermentativen Prozessen im Vormagen unterstützend wirken.

Die mikrobiologische Starterkultur kann zum Beispiel Bakterien, zum Beispiel und ohne Beschränkung, Milchsäurebakterien und/oder Pilze (insbesondere andere als Hefe) aufweisen.

Insbesondere ein Hefepilz oder eine Hefepilzzubereitung kann weiterhin Kohlehydrate, Fett und Eiweiß enthalten, kann also als vielfältiger Energielieferant dienen. Weiterhin ist aufgrund des enthaltenen Eiweißes ein zusätzlicher Bindeeffekt möglich.

Ein ausreichendes Maß an vorteilhaften Stoffwechselprozessen im Vormagen eines Wiederkäuers ist von einer ausreichenden Zufuhr von Rohfasern entscheidend abhängig. Ein solcher vorteilhafter Stoffwechselprozess ist insbesondere die Fermentation von Kohlenhydraten, insbesondere von Zellulose, durch Mikroorganismen. Durch mikrobiologische Starterkulturen verstärken sich solche vorteilhaften Stoffwechselprozesse und/oder setzen ein. Durch Hefe und/oder mikrobiologische Starterkultur kann die Verstoffwechselung der Rohfaser katalysiert werden. Starterkulturen oder Hefe dienen hier vornehmlich als Katalysatoren für die physiologische mikrobiologische Fermentation.

Ein krankes Tier, beispielsweise eine Kuh, hört üblicherweise auf zu fressen, wodurch die Zuführung von Ballaststoffen in den Vormagen gestoppt wird. Hierdurch ergibt sich ein Rohfaserdefizit, was zu Fermentationsstörungen im Vormagen führt, die sich beispielsweise in einer Übersäuerung (Acidose) bemerkbar macht. Der erfindungsgemäße Bolus dient der Zuführung von notwendiger Rohfaser. Ist der Zustand im Vormagen jedoch bereits gestört, wird eine gewisse Zeit benötigt, bis die zugeführte Rohfaser verstoffwechselt wird. Durch die Zugabe von Hefe und/oder mikrobiologischen Starterkulturn in den Bolus werden diese mit dem Bolus in den Vormagen eingeführt und können dort die mikrobiologische Flora verbessern und die Stoffwechselprozesse im Vormagen schneller in Gang bringen, sodass der gestörte Zustand schneller verbessert werden und eine Gesundung schneller erreicht werden kann.

In der gleichzeitigen Zufuhr von strukturwirksamer Rohfaser und mikrobiologischen Starterkulturen und/oder Hefen liegt daher eine besonders vorteilhafte Kombination verschiedener Wirkmechanismen. Es liegt insofern ein Synergismus vor, als dass die mikrobiologischen Starterkultur und/oder Hefe mit der Rohfaser zusammenwirkt um letztere zu verstoffwechseln.

Zusätzlich ist eine tiergerechte Strukturwirksamkeit der Rohfaser zur Durchführung der vorteilhaften Stoffwechselprozesse vorteilhaft. Strukturwirksamkeit heißt insbesondere, dass die Partikellänge und -härte eine vorteilhafte mechanische Wirkung auf den Vormagen hat und diesen zur Kontraktion anregt. Der Wiederkäuer wird hierdurch zum Wiederkauen angeregt.

Insbesondere kann der mindestens eine genannte Bestandteil, der ausgewählt ist aus der Gruppe bestehend aus Hefe und/oder mikrobiologischen Starterkulturen, mit dem mindestens einen Bindemittel oder/und dem Gemenge aus Rohfaser und mindestens einem Bindemittel oder/und mit mindestens einem anderen Bestandteil des Bolus vermischt sein. Dies ist einerseits hinsichtlich eines Herstellungsprozesses des Bolus von Bedeutung, denn der Herstellungsprozess ist durch ein bloßes Hinzufügen und Mischen mit dem mindestens einen genannten Stoff besonders einfach. Andererseits ergibt sich der Vorteil, dass der mindestens eine genannte Stoff sich mit dem mindestens einen Bindemittel in zeitlich kontinuierlicher Art und Weise, entsprechend dem Auflösungsvorgang des mindestens einen Bindemittels, im Vormagen freisetzen kann. Die zeitlich kontinuierliche Art und Weise der Freisetzung kann der Tiergesundheit förderlich sein.

Ebenso kann der genannte Stoff selbst Teil des mindestens einen Bindemittels sein oder in einem Bestandteil des mindestens einen Bindemittels enthalten sein. Die im vorherigen Absatz beschriebenen Vorteile ergeben sich analog.

Die Hinzufügung von mindestens einem bereits erwähnten Spurenelement und/oder einem bereits erwähnten Vitamin und/oder einem bereits erwähnten gesundheits- und/oder verdauungsfördernden Mittel kann je für sich isoliert in den Bolus erfolgen, oder, in einer besonders vorteilhaften Ausführungsform, zusätzlich zu Hefe und/oder mikrobiologischen Starterkulturen. Wie bereits erwähnt, dienen Hefen und mikrobiologische Starterkulturen vorteilhaft dazu, das Verstoffwechseln zugegebener Rohfaser im Vormagen zu beschleunigen.

Ein mikrobiologisches Ungleichgewicht des Vormagens, das insbesondere eine Übersäuerung des Vormagens zur Folge haben kann, ist in vielen Fällen eine Folge einer Erkrankung oder eine Voraussetzung zum Entstehen weiterer Erkrankungen des Wiederkäuers (zum Beispiel Entzündungen der Lunge und/oder anderer Körperteile). Eine Wiederherstellung einer gesunden Vormagenfunktion dient zur Beförderung der Heilung einer bereits vorhandenen Erkrankung oder ist Voraussetzung zur Prävention oder Heilung weiterer Erkrankungen, die erst nach der Störung der Vormagenfunktion auftreten.

Zur Behandlung einer bereits vor Störung der Vormagenfunktion vorhandenen Krankheit, oder einer danach entstandenen Krankheit, kann dem Bolus als weiterer Bestandteil als gesundheitsförderndes Mittel insbesondere eine pharmakologisch wirksame Substanz, beispielsweise ein Medikament, zugegeben werden. Zur Prävention kann ein anderweitiges gesundheitsförderndes Mittel zugegeben werden, beispielsweise ein Kräuterauszug oder Kräuterextrakt.

Ein Bolus, der neben Hefe und/oder mikrobiologische Stadtkultur mindestens eine solche weitere Substanz enthält, übernimmt daher mindestens eine Doppelfunktion in der Wirkweise, es ergibt sich ein Applikationssynergismus hinsichtlich Wiederherstellung der Vormagenfunktion und Heilung oder Prävention einer Krankheit.

Weiterhin ist die Gabe von mehreren Wirkstoffen in einer Anwendung des Bolus im Hinblick auf den Tierschutz förderlich: Der Wiederkäuer braucht eine für ihn möglicherweise unangenehme oder schmerzhafte Behandlung zum Beispiel mittels Spritzen seltener zu ertragen, wenn eine Kombination von Wirkstoffen im Bolus vorhanden ist, mehrere Wirkstoffe also gleichzeitig verabreicht werden können.

In einer weiteren vorteilhaften Ausgestaltung ragt Rohfaser anteilig aus der Oberfläche des Bolus heraus, also anteilig über die Grundgestalt des Bolus hinaus. Es ergibt sich also hieraus eine unregelmäßige, nicht flächige Außenkontur des Bolus. Hierdurch ist die Zersetzung des Bolus im Vormagen erleichtert, da die Angriffsfläche für zersetzende Flüssigkeiten erhöht ist.

Weiterhin wird ein Herstellungsverfahren des Bolus vorgeschlagen, wobei Rohfaser und das mindestens eine Bindemittel vermengt und zu einem Bolus geformt werden.

Mit dem Verfahren kann ein Bolus gemäß jeder der vorangehend beschriebenen Ausführungsformen hergestellt werden. Zuvor anhand eines Bolus verfahrensmäßig offenbarte Merkmale können Bestandteil eines erfindungsgemäßen Verfahrens sein. Zuvor genannte strukturelle Merkmale können in dem Verfahren angewandt oder damit erzeugt werden.

Das Vermengen und/oder das Formen kann in einer weiteren Ausgestaltung des Herstellungsverfahrens unter Druck- und/oder unter Temperatureinwirkung stattfinden.

Dem Gemenge aus Rohfaser und dem mindestens einen Bindemittel können weitere Komponenten oder Bestandteile des Bolus zu jedem Zeitpunkt des Herstellungsverfahrens beigefügt werden.

Hierbei kann eine Vermengung der Komponenten oder Bestandteile unter mechanischer Einwirkung, zum Beispiel mithilfe eines motor- oder muskelbetriebenen oder mittels eines hydraulisch oder pneumatisch arbeitenden Misch- und/oder Press- und/oder Formgeräts, geschehen. Der Vermengungsvorgang kann unter Einwirkung einer Temperatur, die sich von der Umgebungstemperatur unterscheidet, insbesondere höher ist als die Umgebungstemperatur, stattfinden.

Beim Formvorgang kann das Gemenge aus Rohfaser und dem mindestens einen Bindemittel und/oder weiteren Komponenten oder Bestandteilen unter mechanischer Einwirkung, zum Beispiel mithilfe eines motor- oder muskelbetriebenen oder mittels eines hydraulisch oder pneumatisch arbeitenden Press- und/oder Formgeräts, geschehen. Der Formvorgang kann unter Einwirkung einer Temperatur, die sich von der Umgebungstemperatur unterscheidet, insbesondere höher ist als die Umgebungstemperatur, stattfinden.

Im Zuge des Herstellungsverfahrens kann außerdem mindestens ein Mischvorgang des Gemenges mit weiteren Komponenten oder Bestandteilen durchgeführt werden. Auch kann mindestens ein separater Mischvorgang der weiteren Komponenten vor dem Mischvorgang mit dem Gemenge durchgeführt werden. Die hieraus erhaltene Mischung der weiteren Komponenten kann dann dem Gemenge in jeglicher Form und zu jeglichem Zeitpunkt hinzugefügt werden.

Ein Ausführungsbeispiel der Erfindung wird nun unter Bezugnahme auf die beigefügte Zeichnung beschrieben. Die einzelnen Figuren der Zeichnung zeigen:
- Fig. 1: einen erfindungsgemäßen, kapselförmigen Bolus, der ein Gemenge aus Rohfaser und mindestens einem Bindemittel aufweist, von außen in Längsrichtung;
- Fig. 2: einen Längsschnitt durch den in Fig. 1 gezeigten Bolus, wobei ein Randbereich durch eine kreisförmige Umrandung markiert ist;
- Fig. 3: den Randbereich des in Fig. 2 gezeigten Längsschnitts durch den Bolus, wobei auch umliegende Teilbereiche außerhalb der Markierung dargestellt sind;
- Fig. 4: den erfindungsgemäßen, kapselförmigen Bolus (1), der in Fig. 1-3 gezeigt wird, in einer handelsüblichen Eingabehilfe vor dem Eingabevorgang;
- Fig. 5: die handelsübliche Eingabehilfe, die bereits in Fig. 4 gezeigt ist, nach dem Eingabevorgang.

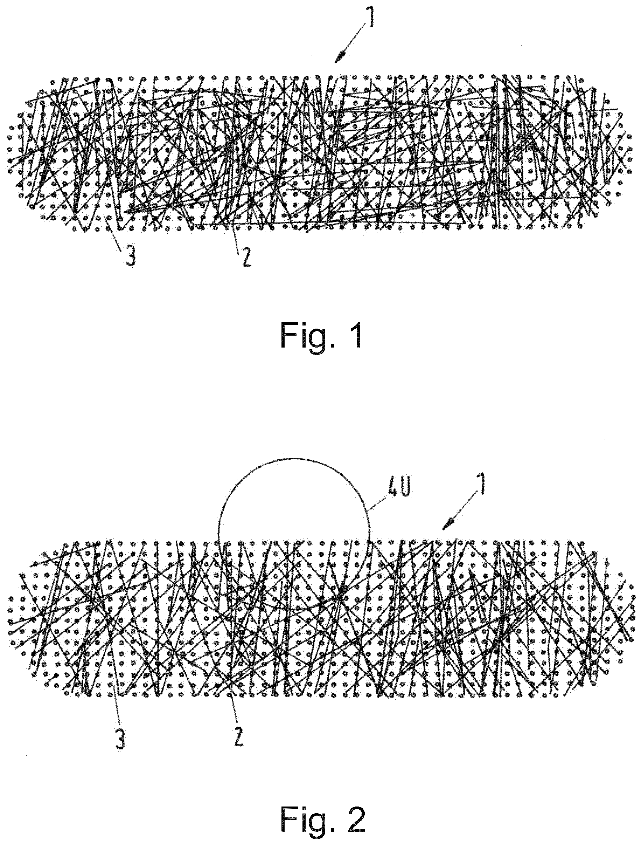

Der Bolus 1, der die dreidimensionale Form einer Kapsel besitzt, also im Querschnitt kreisflächig ist und an den Enden halbkugelartig abgerundet ist, besteht aus einem Gemenge aus Rohfaser 2 und mindestens einem Bindemittel 3. Dies ist in Fig. 1 schematisch dargestellt, wobei die Rohfaser 2 in Form von dunklen Strichen gezeigt ist und das Bindemittel 3 als gepunkteter, kontinuierlicher Bereich. Die Rohfaser 2 ist aufgrund des beschriebenen Herstellungsverfahrens dicht gepackt. Zwischen den einzelnen Bestandteilen der Rohfaser 2 befindet sich das Bindemittel 3 und übt seine Funktion aus, die Bestandteile der Rohfaser 2 zu fixieren und miteinander zu verbinden. In der Realität ist die Dichte der Rohfaser 2 vorzugsweise höher als in den Figuren der Übersichtlichkeit halber gezeigt.

In Fig. 2 ist ein Längsschnitt durch den Bolus 1 dargestellt. Die Bereiche, in denen sich das Bindemittel 3 befindet, sind zwischen den dunklen Strichen, die die Rohfaser darstellen, zu erkennen. Weiterhin ist ein Randbereich des Bolus 1 durch eine kreisförmige Umrandung 4U markiert.

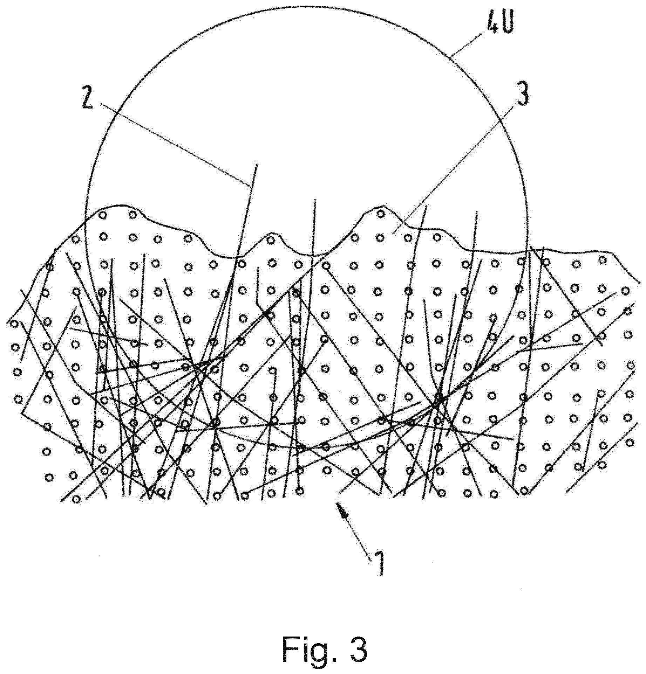

In Fig. 3 ist der Randbereich des Bolus 1, der in Fig. 2 durch die kreisförmige Umrandung 4U dargestellt ist, vergrößert dargestellt. Deutlich zu erkennen ist hierbei, dass die Außenkontur des Bolus 1 nicht vollständig und nicht überall exakt gerade verläuft, auch wenn dies in der Fig. 2 der Einfachheit halber so dargestellt ist bzw. so aussieht. Es ist zu erkennen, dass einzelne Bestandteile der Rohfaser 2 aus der Oberfläche, die durch das zusammenhängende Bindemittel 3 gebildet wird und durch eine dünne, dunkle Konturlinie dargestellt ist, herausragen. Auch ist die Außenkontur des Bolus 1, gebildet durch die einzelnen Bestandteile der Rohfaser und das Bindemittel, unregelmäßig und nicht völlig glatt. Eine derartige Außenkontur liegt in der Natur der verwendeten Bestandteile des Bolus 1 und auch in der Natur des Herstellungsverfahrens.

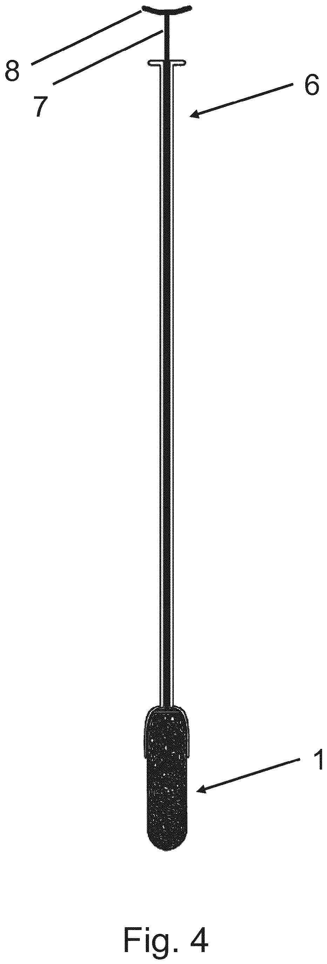

In Fig. 4 ist der Bolus 1, eingesetzt in einer handelsüblichen Eingabehilfe 6 dargestellt. Wird der Druckbolzen 7 oben an der Eingabehilfe 6 durch Druck auf das Handstück 8 betätigt, wird der Bolus 1 abgestoßen. Hierbei befindet sich die Eingabehilfe 6 im Maul- und Rachenbereich und in der Speiseröhre des Tieres.

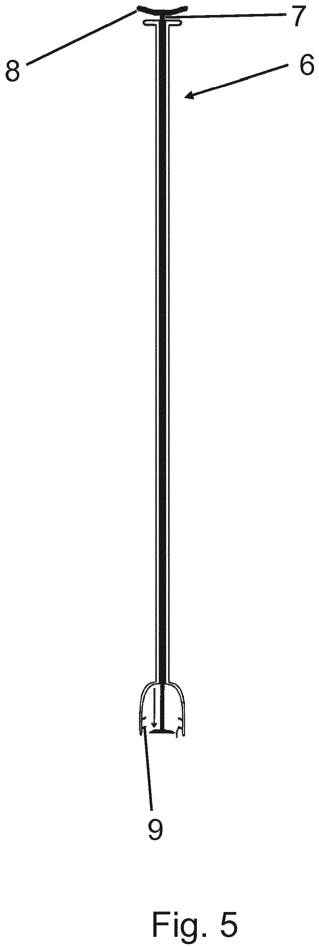

In Fig. 5 ist die Eingabehilfe 6 nach Abstoßen des Bolus 1 gezeigt. Das Handstück 8 ist betätigt worden. Der Bolus 1, der durch klammerartige Haken 9 in der Eingabehilfe 6 gehalten wurde, ist abgestoßen.

### Bezugszeichenliste

- 1: Bolus
- 2: Rohfaser
- 3: Bindemittel
- 4U: kreisförmige Umrandung
- 6: Eingabehilfe
- 7: Druckbolzen
- 8: Handstück
- 9: klammerartige Haken

## Patentansprüche

1. Bolus (1) zur Eingabe in einen Vormagen eines Wiederkäuers, wobei der Bolus (1) ein Gemenge aus Rohfasern (2) und mindestens einem Bindemittel (3) aufweist, wobei das Gemenge aus Rohfasern (2) und dem mindestens einen Bindemittel (3) einen Anteil von mindestens 20 % des Volumens des Bolus (1) ausmacht und wobei die Rohfaser (2) den Vormagen durch mechanische Reize an den Wänden des Vormagens stimuliert.

2. Bolus (1) nach Anspruch 1, wobei Rohfasern (2) einen Anteil von mindestens 20 % des Volumens des Bolus (1) ausmachen.

3. Bolus (1) nach Anspruch 1 oder 2, wobei der Bolus (1) im Wesentlichen aus dem Gemenge aus Rohfasern (2) und dem mindestens einen Bindemittel (3) besteht.

4. Bolus (1) nach einem der vorangehenden Ansprüche, wobei das mindestens eine Bindemittel (3) hydrophobe Eigenschaften aufweist.

5. Bolus (1) nach einem der der vorangehenden Ansprüche, wobei das mindestens eine Bindemittel (3) eine Substanz aufweist, die ausgewählt ist aus der Gruppe bestehend aus Fetten, Proteinen, Kohlehydraten, Salzen, synthetischen hochmolekularen Stoffen und/oder Mineralstoffen und deren Verbindungen.

6. Bolus (1) nach einem der der vorangehenden Ansprüche, wobei die Rohfaser (2) Pflanzenteile aufweist oder aus Pflanzenteilen besteht.

7. Bolus (1) nach Anspruch 6, wobei die Pflanzenteile Heu und/oder Stroh sind.

8. Bolus (1) nach einem der vorangehenden Ansprüche, wobei die Länge der Rohfasern (2) 3 cm bis 8 cm beträgt.

9. Bolus (1) nach einem der vorangehenden Ansprüche, wobei der Bolus (1) als weiteren Bestandteil eine Substanz enthält, die ausgewählt ist aus der Gruppe bestehend aus Hefe und/oder mikrobiologischen Starterkulturen.

10. Bolus (1) nach einem der vorangehenden Ansprüche, wobei der Bolus (1) als weiteren Bestandteil mindestens ein Spurenelement enthält.

11. Bolus (1) nach einem der vorangehenden Ansprüche, wobei der Bolus (1) als weiteren Bestandteil ein Vitamin enthält.

12. Bolus (1) nach einem der vorangehenden Ansprüche, wobei der Bolus (1) als weiteren Bestandteil mindestens ein gesundheits- und/oder verdauungsförderndes Mittel, insbesondere eine pharmakologisch wirksame Substanz, enthält.

13. Bolus (1) nach einem der Ansprüche 9-12, wobei der weitere Bestandteil mit dem mindestens einen Bindemittel (3) und/oder dem Gemenge aus Rohfasern (2) und dem mindestens einem Bindemittel (3) und/oder mit mindestens einem anderen, weiteren Bestandteil des Bolus (1) vermischt ist.

14. Bolus (1) nach einem der vorangehenden Ansprüche, wobei Rohfaser (2) anteilig aus der Oberfläche des Bolus herausragt.

15. Verfahren zur Herstellung eines Bolus (1) nach einem der Ansprüche 1-14, wobei Rohfaser (2) und das mindestens eine Bindemittel (3) vermengt und zu einem Bolus (1) geformt werden.

16. Verfahren nach Anspruch 15, wobei das Vermengen und/oder das Formen unter Druck- und/oder Temperatureinwirkung stattfindet/stattfinden.

## Claims

1. Bolus (1) for administration into a forestomach of a ruminant, wherein the bolus (1) comprises a mixture of crude fibres (2) and at least one binder (3), wherein the mixture of crude fibres (2) and the at least one binder (3) accounts for a proportion of at least 20% of the volume of the bolus (1) and wherein the crude fibres (2) stimulate the forestomach by means of mechanical stimuli on the walls of the forestomach.

2. Bolus (1) according to Claim 1, wherein crude fibres (2) account for a proportion of at least 20% of the volume of the bolus (1).

3. Bolus (1) according to Claim 1 or 2, wherein the bolus (1) substantially consists of the mixture of crude fibres (2) and the at least one binder (3).

4. Bolus (1) according to any of the preceding claims, wherein the at least one binder (3) has hydrophobic properties.

5. Bolus (1) according to any of the preceding claims, wherein the at least one binder (3) comprises a substance selected from the group consisting of fats, proteins, carbohydrates, salts, synthetic high-molecular-weight substances and/or minerals and compounds thereof.

6. Bolus (1) according to any of the preceding claims, wherein the crude fibres (2) comprise plant parts or consist of plant parts.

7. Bolus (1) according to Claim 6, wherein the plant parts are hay and/or straw.

8. Bolus (1) according to any of the preceding claims, wherein the length of the crude fibres (2) is 3 cm to 8 cm.

9. Bolus (1) according to any of the preceding claims, wherein the bolus (1) contains, as further constituent, a substance selected from the group consisting of yeast and/or microbiological starter cultures.

10. Bolus (1) according to any of the preceding claims, wherein the bolus (1) contains, as further constituent, at least one trace element.

11. Bolus (1) according to any of the preceding claims, wherein the bolus (1) contains, as further constituent, a vitamin.

12. Bolus (1) according to any of the preceding claims, wherein the bolus (1) contains, as further constituent, at least one health-promoting and/or digestive agent, more particularly a pharmacologically active substance.

13. Bolus (1) according to any of Claims 9-12, wherein the further constituent is mixed with the at least one binder (3) and/or the mixture of crude fibres (2) and the at least one binder (3) and/or with at least one other further constituent of the bolus (1).

14. Bolus (1) according to any of the preceding claims, wherein crude fibres (2) proportionally protrude from the surface of the bolus.

15. Process for producing a bolus (1) according to any of Claims 1-14, wherein crude fibres (2) and the at least one binder (3) are mixed and are shaped to give a bolus (1) .

16. Process according to Claim 15, wherein the mixing and/or the shaping take(s) place under the action of pressure and/or heat.

## Revendications

1. Bolus (1) destiné à être administré dans un pré-estomac d'un ruminant, le bolus (1) comprenant un mélange de fibres brutes (2) et d'au moins un liant (3), le mélange de fibres brutes (2) et de l'au moins un liant (3) représentant une proportion d'au moins 20 % du volume du bolus (1) et les fibres brutes (2) stimulant le pré-estomac par des irritations mécaniques des parois du pré-estomac.

2. Bolus (1) selon la revendication 1, dans lequel les fibres brutes (2) représentent une proportion d'au moins 20 % du volume du bolus (1).

3. Bolus (1) selon la revendication 1 ou 2, dans lequel le bolus (1) est essentiellement constitué par le mélange de fibres brutes (2) et de l'au moins un liant (3).

4. Bolus (1) selon l'une quelconque des revendications précédentes, dans lequel l'au moins un liant (3) présente des propriétés hydrophobes.

5. Bolus (1) selon l'une quelconque des revendications précédentes, dans lequel l'au moins un liant (3) comprend une substance qui est choisie dans le groupe constitué par les matières grasses, les protéines, les hydrates de carbone, les sels, les substances synthétiques de masse moléculaire élevée et/ou les minéraux et leurs composés.

6. Bolus (1) selon l'une quelconque des revendications précédentes, dans lequel les fibres brutes (2) comprennent des parties de plantes ou sont constituées de parties de plantes.

7. Bolus (1) selon la revendication 6, dans lequel les parties de plantes sont du foin et/ou de la paille.

8. Bolus (1) selon l'une quelconque des revendications précédentes, dans lequel la longueur des fibres brutes (2) est de 3 cm à 8 cm.

9. Bolus (1) selon l'une quelconque des revendications précédentes, dans lequel le bolus (1) contient en tant que constituant supplémentaire une substance qui est choisie dans le groupe constitué par les levures et/ou les cultures de départ microbiologiques.

10. Bolus (1) selon l'une quelconque des revendications précédentes, dans lequel le bolus (1) contient en tant que constituant supplémentaire au moins un oligoélément.

11. Bolus (1) selon l'une quelconque des revendications précédentes, dans lequel le bolus (1) contient en tant que constituant supplémentaire une vitamine.

12. Bolus (1) selon l'une quelconque des revendications précédentes, dans lequel le bolus (1) contient en tant que constituant supplémentaire au moins un agent favorisant la santé et/ou la digestion, en particulier une substance pharmacologiquement active.

13. Bolus (1) selon l'une quelconque des revendications 9 à 12, dans lequel le constituant supplémentaire est mélangé avec l'au moins un liant (3) et/ou avec le mélange de fibres brutes (2) et de l'au moins un liant (3) et/ou avec au moins un autre constituant supplémentaire du bolus (1).

14. Bolus (1) selon l'une quelconque des revendications précédentes, dans lequel les fibres brutes (2) dépassent partiellement de la surface du bolus.

15. Procédé de fabrication d'un bolus (1) selon l'une quelconque des revendications 1 à 14, dans lequel des fibres brutes (2) et l'au moins un liant (3) sont mélangés et mis sous la forme d'un bolus (1).

16. Procédé selon la revendication 15, dans lequel le mélange et/ou la mise en forme ont lieu sous l'effet de la pression et/ou de la température.
